# EUROPEAN PATENT APPLICATION

(11) **EP 2 339 343 A1**
(43) Date of publication of application: **29.06.2011**
(21) Application number: 10193964.3
(22) Date of filing: 07.12.2010
(51) Int. Cl.: G01N 33/543, A61K 49/18, B03C 1/02, G03G 9/083, H01F 1/00

(54) **Magnetic particles and method for producing thereof**

(30) Priority: 08.12.2009 JP 2009278896; 13.10.2010 JP 2010230735
(71) Applicant: JNC Corporation, Chiyoda-ku Tokyo (JP)
(72) Inventor: Eguchi, Masaru, Chiba 290-8551 (JP)
(74) Representative: HOFFMANN EITLE

(57) **Abstract**

The invention provides a magnetic particle which can immobilize the intended substance easily and efficiently; and a method for producing thereof. The invention relates to a magnetic particle, comprising an aggregate which comprises a magnetic substance and a compound having an alkyl group, and a gel layer which covers the aggregate, in which the gel layer has a hydrophilic group.

## Description

### FIELD OF THE INVENTION

The present invention relates to a magnetic particle, comprising an aggregate which comprises a magnetic substance and a compound having an alkyl group, and a gel layer covering the aggregate and having a hydrophilic group, and relates to a method for producing thereof.

### BACKGROUND OF THE INVENTION

A micro-size magnetic Reticle is known for isolation and purification of biomaterials. Since it is simple to use as compared with a column process, they are applied to automation of diagnosis/extraction (Non-Patent Literatures 1 and 2).

In addition, a nano-size magnetic particle for cell selection is well known, and wide usefulness of the method of using nano-size magnetic particles and a magnetic column is shown (Patent Literatures 1 and 2).

### Citation List

### Patent Literature

Patent Reference 1: JP10-500492T
Patent Reference 2: JP2003-508073T

### Non-Patent Literature

Non-Patent Literature 1: Mamma Kawaguchi, "Production of Magnetic Substance-Containing Polymer Particles", Monthly Bioindustry, CMC Publishing, August 2004
Non-Patent Literature 2: Kimimichi Obata, "Automatic Nucleic Acid Extraction Apparatus with Magnetic Particles", BME, 12(2), 1998, pp. 15-24

### SUMMARY OF THE INVENTION

Since a micro-size magnetic particle has a hard surface layer, the hard surface layer would have some negative influence on the substance at the time of collecting a delicate substance. In addition, when a nano-size magnetic particle is separated using a magnet, it has a fault in that it takes a long time for magnetic separation since it is influenced by Brownian motion. Furthermore, when a magnetic column is used in separation of a micro-size magnetic particle or a nano-size magnetic particle, it causes a problem of serious economic burden since cost of column is high.

The invention has been made for solving the above-mentioned problems. The object is to provide a magnetic particle which can purify the intended substance significantly in a much simpler manner, much more inexpensively and efficiently, as compared with an existing magnetic particle.

For solving the above-mentioned problems, the present inventors have assiduously studied. As a result, the inventors have found that a magnetic particle comprising an aggregate which comprises a magnetic substance and a compound having an alkyl group, and a gel layer which is formed on the surface of the aggregate, has excellent reactivity and can readily immobilize protein, amino acid, peptide, sugar and sugar chain. On the basis of this finding, the inventors have completed the invention.

### BRIEF DESCRIPTION OF THE DRAWINGS

Fig. 1 is a figure showing the result of electrophoresis using SDS-PAGE.
Fig. 2 is a figure showing the result of electrophoresis using SDS-PAGE.
Fig. 3 is a figure showing the relation between IgG isolation rate and time.
Fig. 4 is a figure showing the result of electrophoresis using SDS-PAGE.
Fig. 5 is a figure showing the result of electrophoresis using SDS-PAGE.

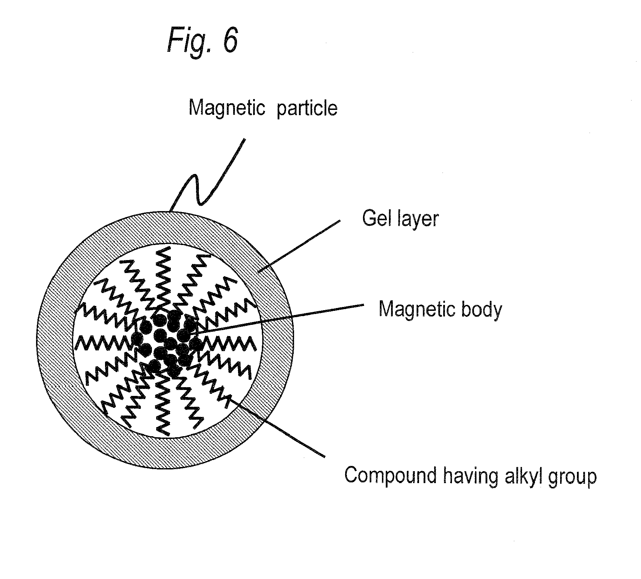

Fig. 6 is a schematic illustration of a magnetic particle comprising a gel layer.
Fig. 7 is a figure showing the result of fluorescent microscopy observation.
Fig. 8 is a schematic illustration showing separation of streptavidin-labeled particles from a mixed solution of FITC-labeled particles and streptavidin-labeled particles.
Fig. 9 is a figure showing the result of microscopy observation.
Fig. 10 is a figure showing the result of fluorescent microscopy observation.
Fig. 11 is a figure showing the result of fluorescent microscopy observation.
Fig. 12 is a figure showing the result of microscopy observation.
Fig. 13 is a figure showing the result of magnetic separation from concentrated tap water and concentrated river water.
Fig. 14 is a figure showing the result of fluorescent microscopy observation.

### DETAILED DESCRIPTION OF THE INVENTION

Specifically, the invention relates to the followings [1] to [16]:
[1] A magnetic particle, comprising an aggregate which comprises a magnetic substance and a compound having an alkyl group, and a gel layer which covers the aggregate, wherein the gel layer has a hydrophilic group;
[2] The magnetic particle according to the above [1], having a mean particle size of from 0.9 nm or more to less than 10000 nm;
[3] The magnetic particle according to the above [1] or [2], wherein the compound having an alkyl group is a compound which does not have a benzene ring and can bind to an iron ion;
[4] The magnetic particle according to the above [3], wherein the compound having an alkyl group is a fatty acid;
[5] The magnetic particle according to the above [4], wherein the fatty acid is oleic acid;
[6] The magnetic particle according to any one of the above [1] to [5], wherein the hydrophilic group is an epoxy group;
[7] The magnetic particle according to any one of the above [1] to [6], wherein the gel layer is a crosslinked body obtained from a polymerizable monomer and a crosslinking monomer;
[8] The magnetic particle according to the above [7], wherein the polymerizable monomer is glycidyl methacrylate and poly(ethylene glycol) methyl ether methacrylate, and the crosslinking monomer is polyethylene glycol diacrylate;
[9] The magnetic particle according to any one of the above [1] to [8], which does not emit autofluorescence;
[10] An adsorption material comprising the magnetic particle of any one of the above [1] to [9], and a substance having an affinity to the intended substance;
[11] The adsorption material according to the above [10], wherein the substance having an affinity to the intended substance is avidin, protein A or antibody;
[12] A method for separating an intended substance, comprising at least the following steps (1) and (2) and uses the adsorption material according to the above [10] or [11]:
   (1) a step of preparing a mixed solution of an aqueous solution containing the intended substance and the adsorption material to form a combined body of the intended substance and the adsorption material,
   (2) a step of separating the combined body by a magnetic force from the mixed solution obtained in the step (1);
[13] The method according to the above [12], further comprising the following step (3):
   (3) a step of eluting the intended substance from the combined body separated in the step (2);
[14] A method of wastewater treatment, comprising using the method according to the above [12] or [13];
[15] The wastewater treatment method according to the above [14], wherein at least one of cryptosporidium and Giardia are separated from the wastewater; and
[16] A method for producing magnetic particle, comprising at least the following steps:
   (1) a step of forming an aggregate that contains a magnetic substance and an compound having an alkyl group,
   (2) a step of forming a gel layer having a hydrophilic group on the surface of the aggregate formed in the step (1) by mixing a polymerizable monomer and a crosslinking monomer and then carrying out polymerization and crosslinking.

Since the magnetic particle of the invention comprise a gel layer which covers an aggregate comprising a magnetic substance and a compound having an alkyl group, the particle has excellent reactivity and can readily immobilize a bioactive protein, amino acid, peptide, sugar and sugar chain.

Embodiments of the invention are described below.

### [Magnetic Particles]

The magnetic particle of the invention comprises an aggregate comprising a magnetic substance and a compound having an alkyl group, and a gel layer covering the aggregate and having a hydrophilic group.

The magnetic particle of the invention has preferably a mean particle size of from 0.9 nm or more to less than 10000 nm. When the mean particle size falls within the range, the particles can obtain good dispersibility. In addition, when the mean particle size of the magnetic particle is from 200 nm or more to less than 5000 nm, it is more preferable since separation efficiency, purification efficiency and convenience of the particle to the intended substance are improved. The mean particle size of the magnetic particle may be calculated by measurement using such as a laser zeta potentiometer (ELS-8000 (product name), manufactured by Otsuka Electronics).

The magnetic particle preferably does not emit autofluorescence. When the particle does not emit autofluorescence, it has the advantage that it is easily distinguished from a fluorescence-labeled substance.

The magnetic particle of the invention is not specifically defined in point of shape. The magnetic particle in any form, such as spherical, needle-like or tabular form, can be used. Spherical, oval or granular form is preferable from the viewpoint of good balance between the trappability and the dispersibility and of the excellence in operability when an intended substance is separated from a magnetic particle (adsorption material) immobilized with a substance having an affinity (hereinafter, referred to as "ligand") to the intended substance to be mentioned below.

### [Aggregate comprising magnetic substance and compound having an alkyl group]

Examples of the aggregate to be used in the invention include a magnetic substance and a compound having an alkyl group.

### [Magnetic Substance]

The "magnetic substance" in the invention means a particle having magnetic response (response to magnetic field), and may be any one capable of imparting magnetic response to a magnetic particle when it is prepared using the particle. "Having magnetic response" as referred to herein means that, when an external magnetic field is present, the substance exhibits response to the magnetic field, namely, it is magnetized by the magnetic field or it is adsorbed by a magnet.

The magnetic substance to be used is not specifically limited as long as it is a conventional magnetic substance which exhibits the above-mentioned magnetic response. Examples of the magnetic substance include a particle of magnetite, goethite, nickel oxide, ferrite, cobalt iron oxide, barium ferrite, carbon steel, tungsten steel, KS steel, rare earth cobalt magnet and hematite.

The magnetic substance may be produced, for example, from a polyalcohol and magnetite in accordance with a known conventional method. For example, in JP2005-082538A, a method for producing a magnetic particle using dextran is disclosed.

The magnetic substance is not specifically limited in point of the form thereof. Examples of the form of the magnetic substance include a polyhedral form, such as spherical, oval, granular, tabular, needle-like and cubic form and the like. Spherical, oval and granular forms are preferable since they can readily realize favorable forms.

"Spherical" as referred to herein means a form in which the aspect ratio (ratio of the maximum length to the minimum length measured in all directions) is within a range of from 1.0 or more to 1.2 or less. "Oval" means a form in which the aspect ratio is from more than 1.2 to 1.5 or less. "Granular" means a form with no specific anisotropy as a whole of the form thereof though having a difference in the length thereof depending on the direction, other than a spherical form in which the length is the same in all directions and an oval form in which the length is long only in one direction.

The size of the magnetic substance is not specifically limited. In order to enable magnetic separation, the mean particle size is preferably from 0.005 to 1 µm, more preferably from 0.2 to 0.5 µm. The mean particle size may be calculated by measurement using such as a laser zeta potentiometer (ELS-8000 (product name), manufactured by Otsuka Electronics).

### [Compound having an alkyl group]

The compound having an alkyl group is a compound having an alkyl group but not having a benzene ring. The compound having an alkyl group is preferably a compound capable of binding to or interacting with an iron ion, more preferably a compound capable of binding to an iron ion. The carbon number of the alkyl group in the compound having an alkyl group is preferably from 4 to 24. When the number of carbon atoms of the alkyl group is greater, the compound tends to be more hydrophobic. Accordingly, the carbon number is more preferably from 10 to 20.

Examples of the compound having an alkyl group include fatty acids, alcohols, silicones and fluoroalkyls. The compound having an alkyl group is more preferably fatty acids and alcohols, and even more preferably fatty acids. Examples of the iron ion include iron (II) ion (Fe²⁺) and iron (III) ion (Fe³⁺).

Examples of the fatty acids to be used include saturated fatty acids and unsaturated fatty acids. Example of the saturated fatty acids include butyric acid, valeric acid, caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, arachic acid, behenic acid and lignoceric acid. Example of the unsaturated fatty acids include palmitoleic acid, oleic acid, elaidic acid, vaccenic acid, linolic acid, (9,12,15)-linolenic acid, (6,9,12)-linolenic acid, 10-methyloctadecanoic acid, arachidonic acid. Among them, oleic acid, myristic acid and stearic acid are preferable from the viewpoint of the ability to form a hydrophobic field and of the solubility in methanol.

Any alcohol may be used as long as alcohol has at least one hydroxyl group in one molecule. Examples of the alcohol include butanol, pentanol, hexanol, capryl alcohol, lauryl alcohol, myristyl alcohol, cetyl alcohol, stearyl alcohol, oleyl alcohol and linolyl alcohol. Since the carbon number of the alkyl which constitutes the alcohol is greater, hydrophilicity tends to be lower, namely, hydrophobicity tends to be higher, so as to form a hydrophobic field. Therefore, from this viewpoint, among them, myristyl alcohol, stearyl alcohol and oleyl alcohol are preferred.

### [Gel Layer]

In the magnetic particle of the invention, the gel layer is formed on the aggregate which comprises a magnetic substance and a compound having an alkyl group and thereby covers the aggregate. "Covering" as referred to herein means the condition where the gel layer is formed in the outermost shell of the resulting magnetic particle by covering the outside of the aggregate. The gel layer preferably completely covers the outside of the aggregate, but may incompletely cover as the outside of the aggregate as long as the gel layer and the aggregate do not separate from each other.

The gel layer has a hydrophilic group. The gel layer preferably has a hydrophilic group both on the surface and the inside of the gel layer. In order to allow a ligand to bind to the hydrophilic group of the gel layer, the gel layer preferably has a hydrophilic group on the surface of the gel layer. In the magnetic particle of the invention, the surface of the magnetic particle is composed of the gel layer, and therefore the particle has flexibility. Accordingly, the magnetic particle has the advantage that the reaction efficiency between the intended substance and the ligand immobilized to the particle is good.

"Flexibility" as referred to herein means flexibleness and elasticity. "Having flexibility" means that the particle is free from steric hindrance which depends on the material surface and the particle does not interfere with the interaction (binding constant) between a ligand and a bioactive protein which is one of the intended substance. The flexibility depends on the content of the crosslinking monomer in the formed gel layer, and preferably, the ratio by mass of the crosslinking monomer to the polymerizable monomer is from 0.01% or more by mass to less than 1% by mass, more preferably from 0.1% or more by mass to 0.8% or less by mass.

### [Hydrophilic Group]

Examples of the hydrophilic group include an epoxy group, a carboxyl group, a hydroxyl group, an amino group and a thiol group. An epoxy group and a carboxyl group are preferred from the viewpoint of converting the hydrophilic group to a different functional group after formation of the magnetic particle and from the viewpoint of the binding of the group to the amino group, the carboxyl group and the thiol group of the ligand.

The gel layer is a crosslinked body obtained from a polymerizable monomer and a crosslinking monomer. The gel layer may comprise a polymer of a polymerizable monomer which is not crosslinked with a crosslinking monomer. The gel layer may be formed by previously producing a polymer of a polymerizable monomer followed by crosslinking it with a crosslinking monomer; or may be formed by mixing a polymerizable monomer and a crosslinking monomer and simultaneously carrying out polymerization and crosslinking.

In the invention, the gel layer is preferably produced by mixing a polymerizable monomer and a crosslinking monomer and simultaneously carrying out polymerization and crosslinking. In the invention, the crosslinked body means the gel layer, including not only a condition containing water or the like but also a dry condition.

Examples of the polymerizable monomer include acrylic acid, methacrylic acid, acrylamide, methacrylamide, hydroxymethacrylamide, glycidyl methacrylate, poly(ethylene glycol) methyl ether methacrylate, 2-hydroxyethyl methacrylate and N-(3-aminopropyl)methacrylamide hydrochloride. Among them, a copolymer of glycidyl methacrylate and poly(ethylene glycol) methyl ether methacrylate are preferable from the viewpoint of having a hydrophilic field and of the convertibility into a different functional group after production and the binding to a ligand.

Examples of the crosslinking monomer include N,N'-methylenebisacrylamide, ethylene glycol dimethacrylate and polyethylene glycol diacrylate. Among them, polyethylene glycol diacrylate is preferable from the viewpoint of forming the gel layer.

In the case where a polymer of a polymerizable monomer is produced in advance and then crosslinked with a crosslinking monomer to form the gel layer, the polymer comprising a polymerizable monomer is preferably a copolymer produced by copolymerization of at least two polymerizable monomers. When two polymerizable monomers are used to produce a copolymer, the two polymerizable monomers may be suitably selected.

For example, the first polymerizable monomer may be selected from acrylic acid, methacrylic acid, acrylamide, methacrylamide, hydroxymethacrylamide, glycidyl methacrylate or N-(3-aminopropyl)methacrylamide hydrochloride, and the second polymerizable monomer may be selected from 2-hydroxyethyl methacrylate or poly(ethylene glycol) methyl ether methacrylate; and these are preferably combined. In this case, the content of the second polymerizable monomer to the mass of the entire mixture of the polymerizable monomers is preferably from 1 to 10% by mass, more preferably from 1 to 5% by mass. When the content falls within the range, a particle having high dispersibility can be obtained.

The crosslinked body may be produced by crosslinking the formed polymer comprising the polymerizable monomer with a crosslinking monomer. The amount of the crosslinking monomer to be mixed is preferably from 0.01 to 1% by mass of the mixture of the polymer comprising the polymerizable monomer and the crosslinking monomer, more preferably from 0.02 to 0.4% by mass. When the amount falls within the range, since the water content based on the crosslinking density of the particle may be increased, the level of immobilization of a ligand to the particle may be increased.

In the case where the gel layer is formed by mixing a polymerizable monomer and a crosslinking monomer and simultaneously carrying out polymerization and crosslinking, at least two polymerizable monomers and a crosslinking monomer are preferably mixed to form the layer. When two polymerizable monomers are used, the two polymerizable monomers may be suitably selected.

The first polymerizable monomer may be selected from such as acrylic acid, methacrylic acid, acrylamide, methacrylamide, hydroxymethacrylamide, glycidyl methacrylate or N-(3-aminopropyl)methacrylamide hydrochloride, and the second polymerizable monomer may be selected from 2-hydroxyethyl methacrylate or poly(ethylene glycol) methyl ether methacrylate, and the crosslinking monomer may be selected from such as N,N'-methylenebisacrylamide, ethylene glycol methacrylate or polyethylene glycol diacrylate; and these are preferably combined.

The ratio of each monomer to be used is preferably, relative to the mixture of the polymerizable monomers and the crosslinking monomer, the first polymerizable monomer is from 89.2 to 98.99% by mass, the second polymerizable monomer is from 1 to 10% by mass, and the crosslinking monomer is from 0.01 to 0.8% by mass.

Examples of the polymerization initiator to be used for polymerization of the polymerizable monomer include 2,2-azobisisobutyronitrile, 2,2'-azobis(2-methylpropionamidine) dihydrochloride, benzoyl peroxide and dimethyl 2,2'-azobis(2-methylpropionate).

In the magnetic particle of the invention, the thickness of the gel layer covering the aggregate comprising a magnetic substance and a compound having an alkyl group is not specifically defined. It may be suitably selected depending on the type of the polymerizable monomer and the crosslinking monomer to be used and depending on the thickness of aggregate.

### [Method for Producing Magnetic Particles]

The magnetic particles of the invention may be obtained according to the production method including at least the following steps:
1) a step of forming an aggregate which comprises a magnetic substance and a compound having an alkyl group,
2) a step of forming a gel layer having a hydrophilic group by producing a crosslinked body from a polymerizable monomer and a crosslinking monomer on the surface of the aggregate formed in the step 1).
In the step 2), it is preferable that at least two polymerizable monomers are used, and the formed polymer is preferably a copolymer. Since the polymerizable monomer has a hydrophilic group, the formed gel layer has the hydrophilic group. Therefore, the hydrophilic group exists on the surface of the gel layer.

The condition for the production method of the invention is not specifically limited. Any condition may be suitably selected depending on the type of the magnetic substance, the compound having an alkyl group, the polymerizable monomer and the crosslinking monomer to be used.

As a specific example, the example where a magnetite particle is used as a magnetic substance, sodium oleate is used as a compound having an alkyl group, glycidyl methacrylate and poly(ethylene glycol) methyl ether methacrylate (having the number-average molecular weight of 2080) are used as a polymerizable monomer, polyethylene glycol diacrylate (having the number-average molecular weight of 575) is used as a crosslinking monomer, and 2,2'-azobisisobutyronitrile is used as a polymerization initiator, is described below.

1) Step of forming an aggregate comprising a magnetic substance and a compound having an alkyl group:
   This step is a step of forming an aggregate by binding a magnetic substance to a compound having an alkyl group. First, magnetite is dispersed in a dispersion medium at room temperature (20°C). The amount of magnetite to be added to the dispersion medium is not specifically limited. The amount of magnetite is preferably added so as to give a concentration of from 2 to 20 mg/ml since it is easy to produce a uniform dispersion.

Examples of the dispersion medium include water, aqueous solution prepared by suitably combining water with buffers (such as, various buffers of HEPES (2-[4-(2-hydroxyethyl)-1-piperazinyl]ethanesulfonic acid), MES (2-(N-morpholino)ethane sulfonic acid), phosphoric acid, Tris, and acetic acid, salts (such as, sodium chloride, potassium chloride, calcium chloride, sodium acetate and magnesium sulfate), surfactants (such as, Triton X-100 and NP-40) and alcohols (such as, ethanol, methanol and isopropanol).

Next, sodium oleate is added to the magnetite dispersion solution. The amount of sodium oleate to be added to the magnetite dispersion solution is preferably from 1 to 30% by mass, more preferably from 3 to 5% by mass.

Subsequently, the mixed solution of magnetite and sodium oleate is heated under reflux and then bind magnetite to sodium oleate to obtain an aggregate comprising magnetite and sodium oleate. Regarding the heating condition for the mixed solution, the mixed solution is preferably reacted under reflux at 70 to 80°C for 6 to 12 hours.

Further, the mixed solution containing the aggregate comprising magnetite and sodium oleate is centrifuged to remove excessive sodium oleate and then again dispersed in a dispersion medium. The aggregate comprising magnetite and sodium oleate is obtained by removing the impurities in the above manner.

2) Step of forming a crosslinking body by mixing a polymerizable monomer and a crosslinking monomer and simultaneously carrying out polymerization and crosslinking so as to cover the aggregate comprising magnetite and sodium oleate formed in the step 1):
   This step is a step of forming a gel layer so as to cover the aggregate formed in the step 1). First, polymerizable monomers, glycidyl methacrylate and poly(ethylene glycol) methyl ether methacrylate (having the number-average molecular weight of 2080), and a crosslinking monomer, polyethylene glycol diacrylate (having the number-average molecular weight of 575), are added to a dispersion solution prepared by dispersing the aggregate comprising magnetite and sodium oleate formed in the step 1) in a dispersion medium.

Regarding the ratio of the monomer to be used, it is preferable that glycidyl methacrylate is added so as to give a concentration of from 89.2 to 98.99% by mass, poly(ethylene glycol) methyl ether methacrylate (having the number-average molecular weight of 2080) is added so as to give a concentration of from 1 to 10% by mass and polyethylene glycol diacrylate (having the number-average molecular weight of 575) is added so as to give a concentration of from 0.01 to 0.8% by mass.

A polymerization initiator, 2,2-azobisisobutyronitrile, is added to the aggregate comprising magnetite and sodium oleate and the mixed solution of the polymerizable monomer and the crosslinking monomer. Regarding the amount of 2,2-azobisisobutyronitrile relative to the mixed solution, it is preferably added so as to give a concentration of from 0.05 to 0.5% by mass.

After the addition of the polymerization initiator, this is heated to copolymerize glycidyl methacrylate and poly(ethylene glycol) methyl ether methacrylate (having the number-average molecular weight of 2080) and simultaneously to crosslink the formed copolymer with polyethylene glycol diacrylate (having the number-average molecular weight of 575). The heating condition is preferably at 70 to 80°C and for 6 to 12 hours.

Then, after restoring to room temperature, the resultant is washed with an organic solvent using magnetic separation to give magnetic a particle having a gel layer on its surface. The organic solvent is removed from the resulting magnetic particles using magnetic separation and substituted with water. After mixing and stirring the particle at room temperature for 24 to 48 hours, the gel layer of the particle is swelled and then dried.

Regarding the drying condition, the particle is preferably dried under reduced pressure at 60 to 70°C for 3 to 6 hours. After dried, the water content of the magnetic particle is preferably from 50 to 90% by mass, more preferably from 70 to 85% by mass. When the water content falls within the range, the dispersibility of the magnetic particle can be high.

According to the above-mentioned process, a magnetic particle of the invention which comprises an aggregate comprising magnetite and sodium oleate, and a gel layer having an epoxy group on the surface and covering the aggregate, in which the gel layer comprises a crosslinked body of glycidyl methacrylate, poly(ethylene glycol) methyl ether methacrylate and polyethylene glycol diacrylate.

### [Adsorption Material]

The magnetic particle of the invention can be an adsorption material capable of specifically adsorbing the intended substance.
The intended substance is not specifically limited. The examples include a bioactive protein, an amino acid, a peptide, a sugar and a sugar chain.

The bioactive protein includes a protein which has a substantially same biological activity as that of mammal, especially human bioactive protein and is derived from a natural protein or a protein obtained by a recombinant method. Examples of the protein obtained by a recombinant method include a protein having the same amino acid sequence as that of a natural protein, and a protein in which one or more amino acids is deleted, substituted or added to the amino acid sequence, having the above-described bioactivity.

Examples of the bioactive protein include a polyclonal antibody; a monoclonal antibody; a protein hormone, such as granulocyte colony stimulating factor (G-CSF), growth hormone, insulin, and prolactin; hematopoietic factor, such as granulocyte/macrophage-colony stimulating factor (GM-CSF), erythropoietin (EPO), and thrombopoietin; cytokine, such as IL-1 and IL-6; tissue plasminogen activator (t-PA); urokinase; serum albumin; blood coagulation factor VIII; reptin; stem cell growth factor (SCF); insulin; and parathyroid hormone. However, the bioactive protein is not limited to the above.

The intended substance includes tag-fused protein. The tag-fused protein is a protein into which a label is introduced using genetic engineering technology or the like. For example, the tag-fused protein may be produced by such as introducing a peptide chain having a specific amino acid sequence or a protein having enzymatic activity and/or binding activity to a specific substance as a label, into a part of the intended protein molecule. When a label is introduced into the intended protein, the separation or detection of the protein can be easily carried out.

Examples of the tag for the tag-fused protein include polyhistidine tag (His tag), GST tag, HA tag, C-Myc tag, V5 tag, VSV-G tag, HSV tag, thioredoxin tag, alkali phosphatase tag and phosphorylated protein tag. However, the tag is not limited to the above.

The amino acid is not specifically limited. Examples include cysteine and methionine. The peptide is not specifically limited. Examples include insulin and glutathion.

Examples of the ligand include biotin, avidin glutathion, lectin, antibody, N-(5-amino-1-carboxypentyl)iminodiacetic acid, protein A and protein G. In the invention, biotin may be iminobiotin, and avidin may be streptavidin.

In the case where the ligand is biotin, the adsorption material may bind to the intended biotinated protein via a specific bond to avidin, and may further bind to their antigen, namely, a various type of proteins, using a biotinated antibody. As the adsorption material of the invention, a commercially-available avidinated protein and biotinated protein can be used, and the biotination may be carried out in accordance with a well-known method in the technical field.

In the case where the ligand is glutathion, the adsorption material may specifically bind to a protein containing glutathion-S-transferase (hereinafter, referred to as "GST"). The protein containing GST may be prepared in accordance with a well-known method in the technical field.

In the case where protein A or protein G is used as a ligand, the intended substance is preferably a polyclonal antibody or a monoclonal antibody. As the polyclonal antibody and the monoclonal antibody, IgG is preferable. Human IgG includes subclasses of IgG1, IgG2, IgG3 and IgG4; and mouse IgG includes subclasses of IgG1, IgG2a, IgG2b and IgG3. In the invention, these are more preferably used.

In the case where the intended substance is a bioactive protein having a sugar chain, the origin of the sugar chain is not specifically limited but is preferably a sugar chain which is added in a mammal cell. Examples of the mammal cell include a Chinese hamster ovarian cell (CHO cells), a baby hamster kidney cell (BHK cells), an African green monkey kidney-derived cell (COS cells) and a human-derived cell.

In the case where the intended substance is a monoclonal antibody, the monoclonal antibody may be produced in any method. The monoclonal antibody can be produced from a hybridoma constructed by fusing an antigen-sensitized immunocyte with a myeloma cell in accordance with an ordinary method for fusing cells, basically using a known technique.

The monoclonal antibody is not limited to a monoclonal antibody produced by hybridoma, but also it includes a chimera antibody which is artificially modified for the purpose of depressing the heteroantigenicity to human and the like. In addition, a human antibody produced by a transgenic animal (a genetically-engineered animal into which a specific gene is introduced on an individual level) and by a phage display and the like are also preferred.

The ligand is immobilized to the gel layer of the magnetic particle. An example of binding of a ligand to the magnetic particle of the invention is disclosed in WO01/009141. Biotin is added to a polymerizable functional group, such as methacryl, acryl or the like, to obtain an addition-polymerizable monomer. The obtained monomer is copolymerized with another monomer to produce an adsorption material in which biotin is bound to the gel layer of the magnetic particle of the invention.

On the other hand, an antibody can be bound to the adsorption material using the bond between avidin and biotin by binding avidin to the antibody as a ligand and then mixing with the adsorption material.
In the case where glutathion is used instead of biotin, glutathion S-transferase may be used instead avidin.

The absorption material in which a substance having an affinity to an antibody (for example, Melon^{™} gel, protein A and protein G) is bound to the surface of the gel layer via a hydrophilic group can be obtained by copolymerizing a monomer having a hydrophilic group with another monomer at the time of forming a gel layer of the magnetic particle in accordance with a well-known method in the technical field. An antibody can be bound to the adsorption material by binding an antibody as a ligand to a substance having an affinity to the antibody.

In addition, the adsorption material of the invention can be used for removal of a microorganism by immobilizing an antibody to the microorganism to the adsorption material. Examples of the microorganisms in wastewater include cryptosporidium, Giardia, Escherichia coli, salmonella, Legionella and Helicobacter pylori.

### [Method for Separating Intended Substance]

Using the adsorption material of the invention, the intended substance may be separated in accordance with the method comprising at least the following steps:
(1) a step of preparing a mixed solution of an aqueous solution containing the intended substance and the adsorption material to form a combined body of the intended substance and the adsorption material;
(2) a step of separating the combined body by a magnetic force from the mixed solution obtained in the step (1);
(3) a step of eluting the intended substance from the combined body separated in the step (2).

The steps are described below.
(1) Step of preparing a mixed solution of an aqueous solution containing the intended substance and the adsorption material to form a combined body of the intended substance and the adsorption material:
   This step is a step of mixing a sample containing the intended substance and the magnetic particle to which a ligand is immobilized (adsorption material) to thereby bind the intended substance to the adsorption material using the affinity between the ligand and the intended substance.

The mixing operation is not specifically limited as long as the intended substance and the adsorption material can contact with each other in a suitable buffer. For example, it is sufficient to lightly tumble for stirring or shake a tube which contains a sample containing the intended substance and the adsorption material. Examples of the mixing operation include a mixing operation using a commercially-available voltex mixer or the like.

Examples of the sample containing the intended substance include a culture medium of a mammal cell, such as a CHO cell, containing a bioactive protein, a culture medium in which the culture medium is subjected to a certain treatment such as partial purification or the like, serum, plasma, ascites, lymph and urine.

In the case where the intended substance is a bioactive protein, in accordance with the method for separating the intended substance of the invention, the bioactive protein can be purified from a sample containing the bioactive protein at a high concentration of preferably from 10⁻¹⁸ to 10⁻² mol/L, more preferably from 10⁻⁵ to 10⁻² mol/L, using the magnetic particles. The concentration of the bioactive protein may be determined, by such as Bradford protein assay, BCA protein assay, measurement of absorbance at 280 nm or electrophoresis. The volume of a sample containing a bioactive protein to be mixed with the adsorption material is preferably at least 1 ml, more preferably at least 15 ml. In addition, the volume is preferably at most 50 L, more preferably at most 10 L.

In the case where the intended substance is a bioactive protein, the sample containing a bioactive protein to be prepared in the step (1) is preferably not pre-treated. Examples of the pretreatment for the sample containing a bioactive protein include pretreatment which is necessary for protein purification in a column process. Examples of the pretreatment include cell separation, ammonium sulfate precipitation, caprylic acid precipitation, dextran sulfate precipitation, polyvinyl pyrrolidone precipitation, phenol red removal using active carbon, desalting and buffer exchange (such as gel permeation, dialysis and ultrafiltration). Examples of the method for cell separation include centrifugation, filtration and ultrafiltration.

Since the method for separating the intended substance of the invention does not require pretreatment of the sample containing a bioactive protein, the time for separating a bioactive protein may be shortened. The required time for the pretreatment may differ depending on the type and the amount of the bioactive protein and the required time is generally from 15 to 30 hours. In the case where foreign cells are separated by centrifugation, in general, it takes approximately from 30 minutes to 1 hour. In the case where a sample containing a bioactive protein is serum, it requires ammonium sulfate precipitation and desalting. When the amount of serum is from 15 to 1000 ml, the required time is generally from 30 minutes to I hour. In the case where a sample containing a bioactive protein is a culture supernatant, it optionally requires phenol red removal. When the amount of the culture supernatant is from 15 to 1000 ml, the required time is generally about 1 hour.

(2) Step of separating the combined body by a magnetic force from the mixed solution obtained in the step (1):
   This step is a step of separating the adsorption material to which the intended substance is bound, by a magnetic force from the mixture obtained in the step (1). Specifically, for example, in the case where the intended substance is subjected to bind to the adsorption material in a suitable tube, a magnet is moved closer to the side wall of the tube from the outside to keep the adsorption material near the side wall of the tube. By discarding the supernatant liquid from the tube, the intended substance-bound adsorption material can be separated.

The magnetic force of the magnet or the like to be used for separation of the magnetic particles may differ depending on the strength of the magnetic force of the magnetic particle to be used. The strength of the magnetic force may be suitably selected so as to collect the intended magnetic particle magnetically. The material of the magnet may be a material to constitute the magnetic particle. For example, neodymium magnet (manufactured by Niroku Seisakusho) and the like can be used. More preferably, the magnetic force of the neodymium magnet is at least 3800 gausses.

(3) Step of eluting the intended substance from the combined body separated in the step (2):
   This step is a step of eluting and isolating the intended substance from the combined body of the intended substance and the adsorption material separated through magnetic separation in the step (2). In accordance with the property of the intended substance, the intended substance is separated from the combined body according to a well-known method in the technical field.

Specifically, for example, by adding a suitable eluate to the tube into which the combined body is put, the intended substance is eluted from the combined body. After the elution, a magnet is moved closer to the side wall of the tube from the outside. By keeping the magnetic particles near the side wall of the tube and then collecting supernatant from the tube, the intended substance bound to the adsorption material can be isolated.

The eluate to elute the intended substance is preferably a liquid having the effect of lowering the affinity between the intended substance and the ligand. Examples of the liquid include a buffer containing hydrochloric acid, imidazole, glutathion and biotin. Examples of the buffer include potassium phosphate buffer, sodium phosphate buffer, tris hydrochloride buffer, 1,4-piperazine-diethane-sulfonic acid buffer (hereinafter, referred to as "PIPES buffer"), boric acid buffer and glycine hydrochloride buffer.

Before the intended substance is eluted from the combined body of the intended substance and the adsorption material, the combined body may be washed to remove foreign substances. Specific examples of the washing method include a method in which a combined body of the intended substance and the adsorption material is added to a sodium phosphate buffer containing 0.5% by mass of Tween 20 (Unichema Chemie BV's trade mark, polyoxyethylene sorbitan monolaurate) and redispersed therein. By repeating the above step, the foreign substances incorporated into the combined body by binding via hydrophobic interaction or inclusion can be removed.

As a result of the process of the above-mentioned steps (1) to (3), the intended substance can be purified from the sample containing the intended substance.

Using the adsorption material of the invention enables wastewater treatment by carrying out the process of at least the above-mentioned steps (1) and (2).

### Examples

The invention is described with reference to the following Examples and Comparative Examples; however, the invention is not limited to these Examples.
Magnetic separation means collection of magnetic particles or the like from a liquid by the magnetic force of a magnet or the like. For the magnetic separation, neodymium magnet manufactured by Niroku Seisakusho is used.
Pure water means water having a conductivity of 18 MΩ cm, purified using a pure water making machine manufactured by Millipore, "Direct-Q^{™}".

### Example 1:

### <Method for producing magnetic particles having gel layer on the surface>

To a 200-ml flask, 100 ml of an aqueous mixed solution of ferric chloride hexahydrate (1.0 mol) and ferrous chloride tetrahydrate (0.5 mol) was added, and stirred with a mechanical stirrer (Laboratory Height Power Mixer, manufactured by ASONE). Then, the mixed solution was heated up to 50°C. To the mixed solution, 5.0 ml of 28 % by mass of an aqueous ammonia solution was added dropwisely and stirred for about 1 hour. Through the above process, magnetite having a mean particle size of about 5 nm was obtained. The resulting magnetite was washed three times with pure water, dried and dispersed in methanol to give a concentration of 20 mg/ml. Sodium oleate was added to the magnetite dispersion solution in an amount of 3 g relative to 100 ml of the dispersion solution, and refluxed at 80°C for 12 hours. Excessive sodium oleate was removed by centrifugation (10000 g, 30 minutes, 25°C). By dispersing the residue in methanol again so as to give a concentration of 20 mg/ml, a methanol dispersion of magnetite/sodium oleate composite was obtained.

To 100 ml of the methanol dispersion of magnetite/sodium oleate composite (magnetic particles), 25 g of glycidyl methacrylate, 1 g of poly(ethylene glycol) methyl ether methacrylate (having the number-average molecular weight of 2080) and 0.1 g of polyethylene glycol diacrylate (having the number-average molecular weight of 575) were added and stirred at room temperature for 2 hours. Then, to the obtained mixture, 0.25 g of 2,2'-azobisisobutyronitrile was added and heated at 70°C to react for 6 hours. The resulting reaction mixture was restored to room temperature, then subjected to magnetic separation (with neodymium magnet manufactured by Niroku Seisakusho), and washed two times with methanol to give a methanol dispersion of magnetic particle having a gel layer on its surface. Methanol was removed from the methanol dispersion solution of magnetic particles through magnetic separation (with neodymium magnet manufactured by Niroku Seisakusho), and substituted with pure water. The obtained mixture was stirred by rotation overnight to swell the gel layer. Subsequently, 1 ml of the dispersion was sampled and then subjected to magnetic separation to remove the supernatant. The mass of the magnetic particles containing water were measured, then the particles were dried (at 60°C for 6 hours in a constant-temperature drier, DKN402, manufactured by Yamato Scientific), and the mass of the dried magnetic particles was measured. As a result, the water content of the gel-layer having magnetic particles was 83% by mass.

The mean particle size of the obtained magnetic particles was measured using a laser zeta potentiometer (ELS-8000 (product name), manufactured by Otsuka Electronics). The obtained mean particle size was 525 nm.

### Example 2

### <Isolation of biotinated antibody with streptavidin-immobilized magnetic particles>

### (Preparation of streptavidin-immobilized magnetic particles)

First, 1 ml (20 mg/ml) of the magnetic particles comprising a gel layer prepared in the same manner as in Example 1 were subjected to magnetic separation to remove the supernatant. To the residue, 1 ml of pure water was added and then the magnetic particles were redispersed. By carrying out magnetic separation again, the supernatant was removed. To the residue, 1 ml of 25 mM MES buffer (pH 4.75) was added and the magnetic particles were redispersed. To the obtained solution, 25 µl of streptavidin (10 mg/ml) was added and stirred by rotation for 3 hours. After the resulting solution was subjected to magnetic separation, the supernatant was removed. Then, to the residue, 1 ml of 100 mM TBS (tris-buffered saline) was added and reacted for 1 hour. After magnetic separation, the supernatant was removed. To the resulting magnetic particles, 1 ml of 100 mM PBS (phosphate-buffered saline, pH 7.5, containing 0.01% by mass of BSA) was added and the particles were redispersed. The same operation was repeated once more to obtain streptavidin-immobilized magnetic particles.

### (Isolation of biotinated antibody with streptavidin-immobilized magnetic particles)

To 100 µl of the streptavidin-immobilized magnetic particles, 10 µg of biotinated IgG (Anti-IgG (Fc), Rabbit, Goat-Poly Biotin, manufactured by ROCKLAND) was added and stirred by rotation for 5 minutes. Next, 15 µl of the obtained solution was sampled to be a total sample for electrophoresis using SDS-PAGE (sodium dodecyl sulfonate-polyacrylamide gel electrophoresis). This solution was subjected to magnetic separation for 2 minutes and 15 µl was sampled from the supernatant to be a supernatant (sup) sample for electrophoresis using SDS-PAGE. The supernatant was removed. To the resulting magnetic particles, 1 ml of 100 mM PBS (pH 7.5, containing 0.01% by mass of BSA) was added and then the particles were redispersed. After the same operation was repeated once more, the supernatant was removed. Then, 15 µl of 100 mM PBS was added to the residue to be a precipitation (ppt) sample for electrophoresis using SDS-PAGE.

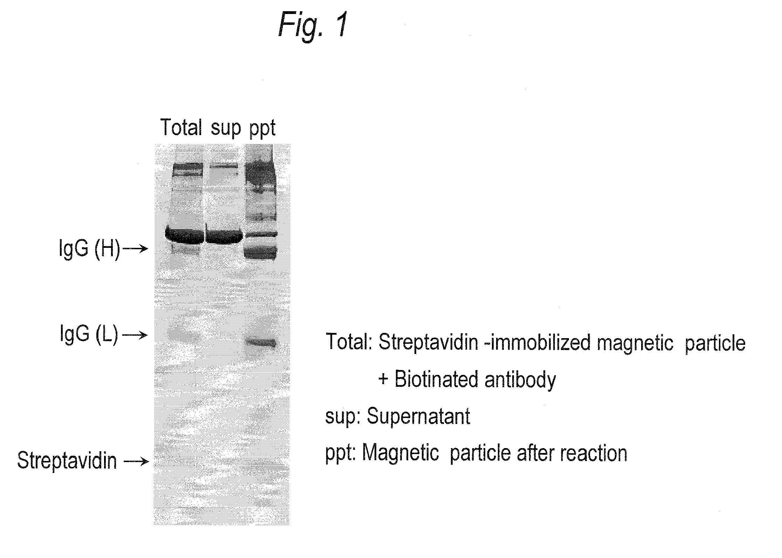

Then, 15 µl of each sample was analyzed by electrophoresis using SDS-PAGE and the results are shown in Fig. 1. As shown in Fig. 1, it is found that the band derived from IgG which existed in the total sample was disappeared in the sup sample and the IgG was bound to the magnetic particles (ppt sample).
From the results, it was found that the biotinated IgG could be efficiently isolated by the streptavidin-immobilized magnetic particles of the invention.

### Example 3:

### <Purification of antibody with protein A-immobilized magnetic particles>

### (Preparation of streptavidin-immobilized magnetic particles)

The magnetic particles comprising a gel layer were prepared in the same manner as in Example 1. Then, 1 ml (20 mg/ml) of the magnetic particles was subjected to magnetic separation to remove the supernatant, To the residue, 1 ml of pure water was added and the magnetic particles were redispersed. After magnetic separation once again, the supernatant was removed. To the residue, 1. ml of 25 mM MES buffer was added and then the magnetic particles were redispersed. To the obtained solution, 25 µl of protein A (10 mg/ml) was added and stirred by rotation for 3 hours. The obtained solution was subjected to magnetic separation to remove the supernatant. Then, to the residue, 1 ml of 100 mM TBS (pH 7.5) was added and reacted for 1 hour. After magnetic separation, the supernatant was removed. To the residue, 1 ml of 100 mM PBS (pH 7.5) added and the magnetic particles were redispersed. The same operation was repeated once more to obtain protein A-immobilized magnetic particles.

### (Isolation of IgG from rabbit and human sera with protein A-immobilized magnetic particles)

To 100 µl of the protein A-immobilized magnetic particles, 10 µl of rabbit or human serum was added and stirred by rotation for 5 minutes with MTR-103 (manufactured by AS ONE). After the supernatant was removed, 1 ml of 100 mM PBS was added to the residue and then the magnetic particles were redispersed. After this solution was subjected to magnetic separation once again, the supernatant was removed and then the magnetic particles were redispersed. After magnetic separation, the supernatant was removed, and 90 µl of 100 mM glycine hydrochloride buffer (pH 3.0) was added to the residue and mixed by shaking for 3 minutes to elute IgG (the first eluate). After magnetic separation, the supernatant was separated and 10 µl of 10 × PBS was added to the separated supernatant to obtain an eluate sample 1.

Again, 90 µl of 100 mM glycine hydrochloride buffer (pH 3.0) was added to the residue and mixed by shaking for 3 minutes to elute IgG (the second eluate). To the separated supernatant, 10 µl of 10 × PBS was added to obtain an eluate sample 1. After magnetic separation, the supernatant was separated and 10 µl of 10 × PBS was added to the supernatant to obtain an eluate sample 2.

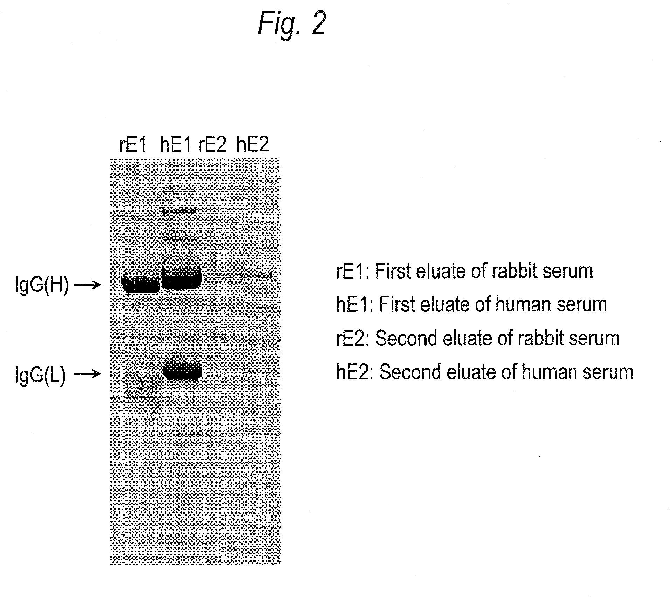

Then, 15 µl of each sample was analyzed by electrophoresis using SDS-PAGE and the results are shown in Fig. 2. As shown in Fig. 2, it was found that IgG could be separated and eluted from any of rabbit serum and human serum using the protein A-immobilized magnetic particles of the invention.

### Example 4:

### <Comparison between protein A-immobilized magnetic particles and protein G-immobilized magnetic particles, and existing products>

### (Isolation of IgG)

Protein A-immobilized magnetic particles and protein G-immobilized magnetic particles were prepared in the same manner as in Example 3. Each 3 mg of the protein A-immobilized magnetic particles and the protein G-immobilized magnetic particles were added to 100 µl of 100 mM PBS containing 200 µg ofIgG Fraction ofAnti-Streptavidin Rabbit (manufactured by ROCKLAND) and stirred by rotation at 25°C for 15 minutes using MTR-103 (manufactured by AS ONE). After magnetic separation for 2 minutes, the supernatant was removed. To the residue, 200 µl of 100 mM PBS-T (PBS solution containing 0.05% by mass of Tween 20) was added and the magnetic particles were redispersed. After magnetic separation once more, the supernatant was removed and the residue was redispersed. The obtained solution was washed further two times to remove the supernatant. To the residue, 45 µl of 100 mM glycine hydrochloride buffer (pH 2.7) was added and mixed by pipetting. After the obtained mixture was vortexed for 5 minutes, the mixture was subjected to magnetic separation and the supernatant was collected. After the residue was again subjected to extraction, the two supernatants were combined. To the combined solution, 8 µl of 1 N NaOH was added to neutralize. The neutralized supernatant was electrophoresed using SDS-PAGE.

The results of electrophoresis were analyzed using CS Analyzer (manufactured by ATTO). The standard curve was created using IgG Fraction of Anti-Streptavidin Rabbit. In addition, the same operation was done with Dynabeads Protein A and G (manufactured by Invitorogen).

As a result of electrophoresis, the protein A-immobilized magnetic particles separated 158.4 µg of IgG; and Dynabeads Protein A separated 36.8 µg of IgG. The protein G-immobilized magnetic particles separated 97.4 µg of IgG; and Dynabeads Protein G separated 27.3 µg of IgG.

From the above results, it was found that the protein A-immobilized magnetic particles and the protein G-immobilized magnetic particles of the invention separated IgG at most 4.3 times and at most 3.6 times, respectively, larger than that Dynabeads Protein G.

### (Isolation of IgG from diluted solution)

Protein A-immobilized magnetic particles were prepared in the same manner as in Example 3. To 50 ml of a dilute solution containing 50 µg of IgG Fraction of Anti-Streptavidin Rabbit (manufactured by ROCKLAND), 1 mg of protein A-immobilized magnetic particles were added and stirred by rotation for 30 minutes. After magnetic separation, the supernatant was removed. To the residue, 1 mL of 100 mM PBS was added and then the magnetic particles were redispersed. After magnetic separation once again, the supernatant was removed and then the magnetic particles were redispersed. After magnetic separation, the supernatant was removed. Then, the magnetic particles were analyzed by electrophoresis using SDS-PAGE.

The results of electrophoresis were analyzed using CS Analyzer (manufactured by ATTO). The standard curve was created using IgG Fraction of Anti-Streptavidin Rabbit. In addition, the same process was done with Dynabeads Protein A (manufactured by Invitorogen).

As a result, 25 µg of IgG was separated using the protein A-immobilized magnetic particles. On the other hand, 6 µg of IgG was separated using Dynabeads Protein A. From the results, it is found that the protein A-immobilized magnetic particles of the invention separated IgG from the dilute solution to a degree of about 4 times larger than that with Dynabeads Protein A.

### (Comparison in reaction speed of magnetic particles with IgG)

Protein A-immobilized magnetic particles were prepared in the same manner as in Example 3. To 500 µl of a PBS solution containing 25 µg of IgG Fraction of Anti-Streptavidin Rabbit (manufactured by ROCKLAND), 10 mg of the protein A-immobilized magnetic particles were added. After reacted for 0.5, 1,5, 10, 20 and 30 minutes, the obtained solution was subjected to magnetic separation. Then, 15 µl of the supernatant was sampled and electrophoresed.

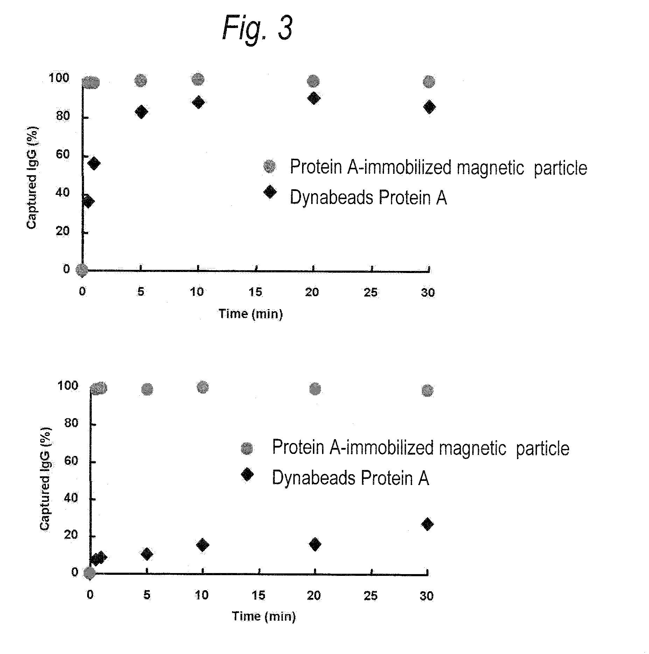

The results of electrophoresis were analyzed using CS Analyzer (manufactured by ATTO). Dynabeads Protein A (manufactured by Invitrogen) were tested in the same manner. Protein G-immobilized magnetic particles were tested in the same manner and compared with the above. The results are shown in Fig. 3.

As shown in the upper graph in Fig. 3, the protein A-immobilized magnetic particles separated almost 100% IgG in about 0.5 minutes. On the other hand, Dynabeads Protein A separated 85% IgG in 30 minutes. As shown in the lower graph in Fig. 3, the protein G-immobilized magnetic particles separated almost 100% IgG in 0.5 minutes. On the other hand, Dynabeads Protein G separated 27% IgG in 30 minutes. From these results, the protein A-immobilized magnetic particles and the protein G-immobilized magnetic particles of the invention have a higher reaction speed to IgG than Dynabeads Protein A and Dynabeads Protein G.

### Example 5:

### <Isolation of IgG2a from 50 ml of hybridoma culture supernatant with protein A-immobilized magnetic particles>

### (Process 1)

To a 300-ml Erlenmeyer flask, 50 ml of hybridoma culture supernatant was added and then further 250 µl of 10 % by mass of Tween 20 was added (final concentration, 0.05% by mass). After the protein A-immobilized magnetic particles prepared in the same manner as in Example 3 (10 mg/ml), 4 mL of the obtained particles were added to the solution and then incubated without bubbling at room temperature for 1 hour with a shaker (90 rpm, MES-1 Minishaker, manufactured by IKA).

### (Process 2)

The obtained solution was uniformly transferred into two 50-ml centrifugal tubes and subjected to magnetic separation at room temperature for 10 minutes. After the supernatant was returned back to the Erlenmeyer flask, 4 ml of protein A-immobilized magnetic particles were added again and then incubated for 1 hour in the same manner.

### (Washing Operation)

To the magnetic particles subjected to magnetic separation in the process 1, 3 ml of PBS-T (PBS containing 0.05% by mass of Tween 20) was added and the particles were well dispersed. The dispersion solution was transferred into two 2-ml, microtubes. After, 1 ml of PBS-T was added to two empty centrifugal tubes, respectively, to wash the wall surface of the tube, 0.5 ml of PBS-T was added to the above 2-ml microtubes. These tubes were set in a magnetic separator (Magnastant-8, manufactured by Magnabeat), left to stand in ice-water for 5 to 10 minutes and then subjected to magnetic separation. After the supernatant was removed, PBS-T was added again to the residue and then the magnetic particles were dispersed. The obtained solution was subjected to magnetic separation for 5 to 10 minutes and the supernatant was removed. After the washing operation was repeated again, the supernatant was removed.

### (Extraction Operation)

To each tube, 450 µl of 100 mM glycine hydrochloride buffer (pH 2.7) was added and mixed well. The microtubes were inserted into a float and set in ice-water. The microtubes inserted with the float were put into a sonicator (S30H Elmasonic, manufactured by Elma) and washed ultrasonically. The ultrasonic washing was carried out for 5 minutes repeating a washing cycle of 10 second for washing and then 10 seconds for pausing. After the magnetic separation, the supernatant was separated and transferred into a fresh microtube. To the microtube, 80 µl of 1 M NaOH added to neutralize. In this manner, purified IgG2a was obtained. The extraction operation was repeated once more. Those separated with the protein A-immobilized magnetic particles in the process 2 were washed and extracted in the same manner.

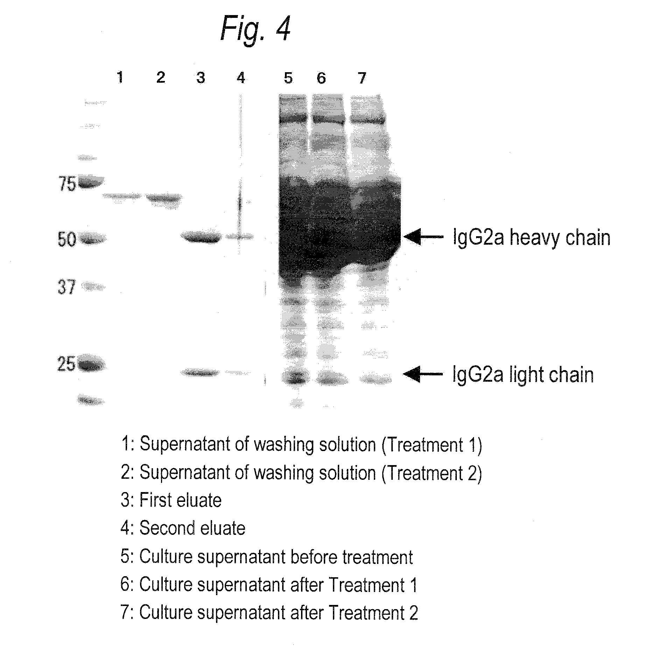

Then, 15 µl of each sample was analyzed by electrophoresis using SDS-PAGE, and the results are shown in Fig. 4.

As shown in Fig. 4, the washed solution of the supernatant had no band derived from IgG, but only the eluted solution had the band derived from IgG. From the results, it was found that the operation with the magnetic particles of the invention enabled specific purification of IgG The isolation rate was 93% and the required time was about 4 hours.

### Example 6:

### <Isolation of His-protein A with AB-NTA-immobilized magnetic particles>

### (Immobilization ofAB-NTA)

In the same manner as in Example 1, 10 ml (20 mg/ml) of magnetic particles comprising a gel layer were prepared and subjected to magnetic separation to remove the supernatant. To the residue, 10 ml of pure water was added and the magnetic particles were redispersed. After magnetic separation once more, 10 ml of 100 mM boric acid buffer (pH 8.5) was added to the residue and the magnetic particles were redispersed. To the obtained solution, 100 mg of AB-NTA free acid (manufactured by DOJIDO) was added and stirred by rotation for 12 hours. After magnetic separation, the supernatant was removed. To the residue, 10 ml of pure water was added and the magnetic particles were redispersed. The same operation was repeated three more times to obtain AB-NTA-immobilized magnetic particles.

### (Formation of composite with nickel)

After 10 ml of the AB-NTA-immobilized magnetic particles were subjected to magnetic separation, the supernatant was removed and the residue was suspended in 10 ml of 1 M of nickel sulfate solution followed by stirring for 6 hours. Subsequently, the obtained solution was subjected to magnetic separation and the supernatant was removed. To the residue, 10 ml of pure water was added and the magnetic particles were redispersed. After the same operation was repeated three more times, the residue was re-suspended in PBS (pH 7.5) and the Ni-NTA magnetic particles were restored therein.

### (Isolation of His-protein A)

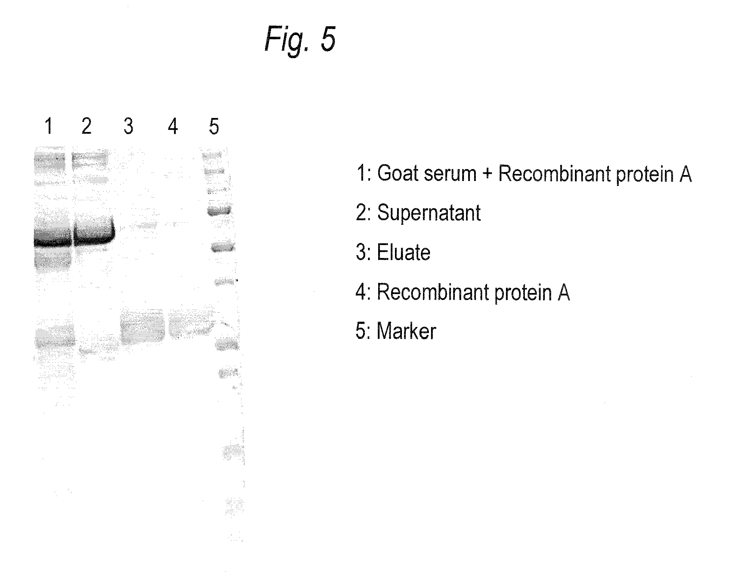

To 10 µl of goat serum containing 100 µg of recombinant-protein A containing His-tag at the N-terminal thereof (manufactured by Funakoshi), 1 mg of Ni-NTA magnetic particles were added. After the obtained solution was reacted for 5 minutes, the supernatant was removed. To the residue, 1 ml of PBS containing 0.1% by mass of Tween 20 was added and the Ni-NTA magnetic particles were redispersed. Similarly, the particles were washed five times with 1 ml of PBS containing 0.1% by mass of Tween 20. After magnetic separation, the supernatant was removed. To the residue, 50 µl of 500 mM imidazole PBS solution was added and then eluted. Then, 15 µl of the supernatant was sampled and analyzed by electrophoresis using SDS-PAGE. The results were shown in Fig. 5.

As shown in Fig. 5, the eluted solution showed a band derived from recombinant-protein A-derived. From the results, it is found that the Ni-NTA magnetic particles of the invention specifically separate a protein containing His-tag.

### Example 7:

### <Evaluation of presence or absence of autofluorescence>

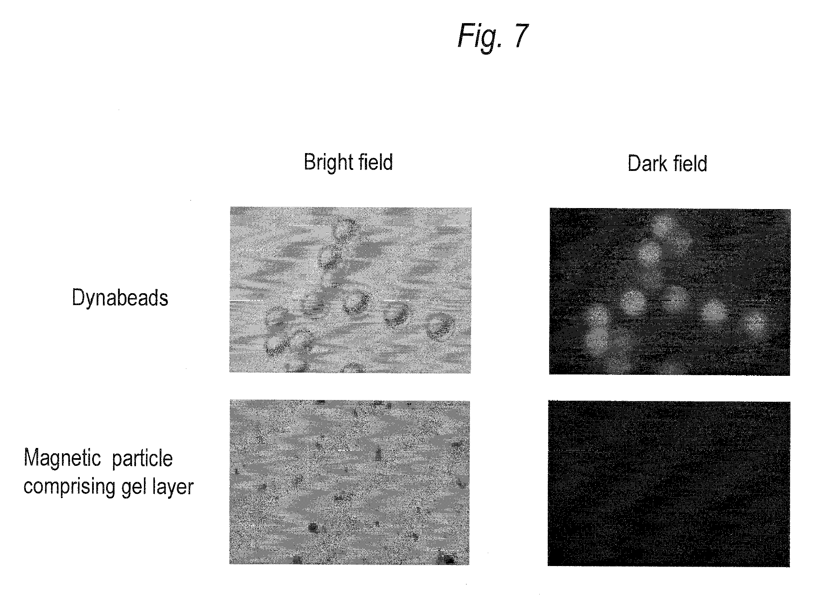

Magnetic particles comprising a gel layer were prepared in the same manner as in Example 1 and observed using a fluorescent microscope (arc light source total reflection fluorescent microscope system IX71-ARCEVA, manufactured by Olympus) in the bright field and the dark field. Dynabeads anti-cryptosporidium (manufactured by Veritas) were observed similarly. The results are shown in Fig. 7.

As shown in Fig. 7, Dynabead exhibited autofluorescence in the dark field, but the magnetic particle having a gel layer did not exhibit autofluorescence. From the results, it was found that the magnetic particles of the invention did not emit autofluorescence.

### Example 8:

### <Selectionof streptavidin-labeled particles from mixture of FITC-labeled particles and streptavidin-labeled particles, with anti-streptavidin antibody-immobilized magnetic particles> (Immobilization of anti-streptavidin antibody to magnetic particles)

Magnetic particles comprising a gel layer were prepared in the same manner as in Example 1 and 1 ml (20 mg/ml) of the obtained particles were subjected to magnetic separation and then the supernatant was removed. To the residue, pure water was added and then the magnetic particles were disperesed. After magnetic separation again, the supernatant was removed. To the residue, 1 ml of 25 mM MES buffer (2-(N-morphalino)ethanesulfonic acid, pH 4.75) was added and then the magnetic particles were disperesed. To the resulting solution, 25 µl of anti-streptavidin antibody (10 mg/mL, Anti-Streptavidin Rabbit, manufactured by ROCKLAND) was added and stirred by rotation for 3 hours. Subsequently, the obtained solution was subjected to magnetic separation and then the supernatant was removed. After 1 ml of 100 mM TBS was added to the residue and reacted for 1 hour. After magnetic separation, the supernatant was removed. To the residue, 1 ml of 100 mM PBS (pH 7.5, containing 0.01% by mass of BSA) was added and the residue was dispersed. The same operation was repeated once more to obtain anti-streptavidin antibody-immobilized magnetic particles.

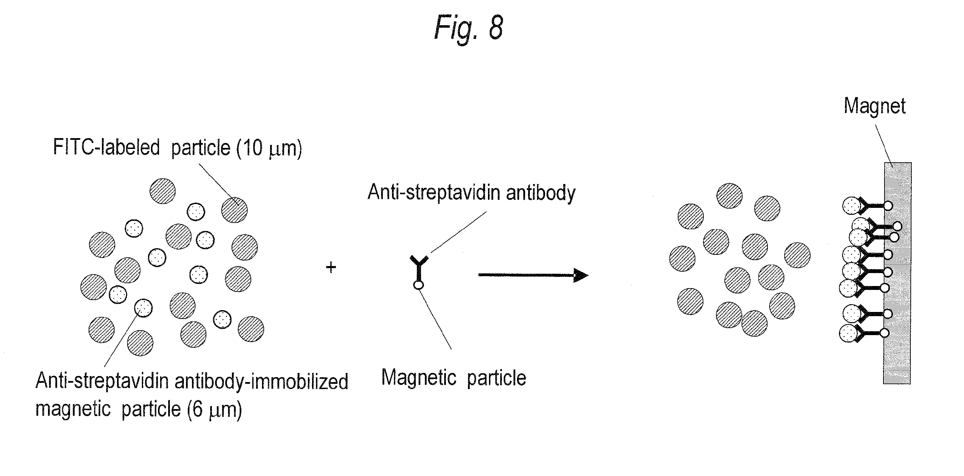

### (Selection of streptavidin labeled particles from mixture of FITC-labeled particles and streptavidin-labeled particles (Fig. 8))

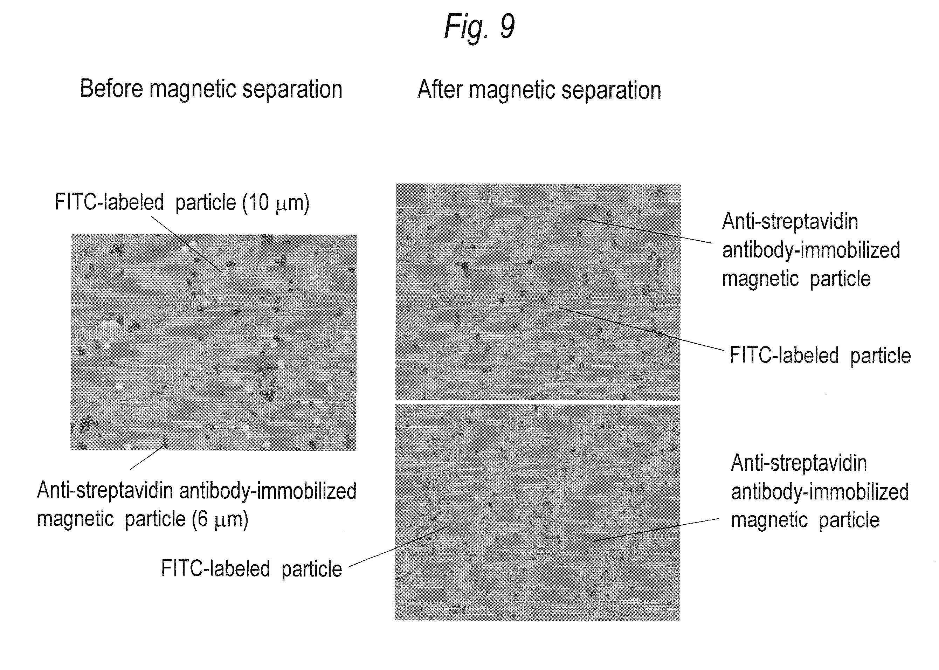

First, 50 µl of the anti-streptavidin antibody-immobilized magnetic particles were added to 100 µl of a mixture prepared by mixing FITC-labeled particles (microparticles based on melamine resin, carboxylate-modified, FITC-marked, manufactured by Fluka, 10 µm, 2.5% by mass) and streptavidin-labeled particles (Streptavidin Microspheres, 6.0 µm, manufactured by Polysciences, 1.36% by mass) in a ratio (by volume) of 1/10, and stirred by rotation for 15 minutes. Subsequently, the obtained solution was subjected to magnetic separation, the supernatant was removed and the magnetic particles were redispersed in 1 ml of PBS. After magnetic separation, the supernatant was removed and the magnetic particles were redispersed in 1 ml of PBS. The same operation was repeated two more times. Subsequently, the obtained solution was observed with a fluorescent microscope and a microscope (IX71, manufactured by Olympus). Fig. 9 shows the results of microscopic observation. As shown in Fig. 9, before the magnetic separation using the magnetic particles of the invention, the number of the FITC-labeled particles was about 20% of the total; but after the magnetic separation, it was about 1.4%.

### (Confirmation of non-specific adsorption of FITC-labeled particles by magnetic particles)

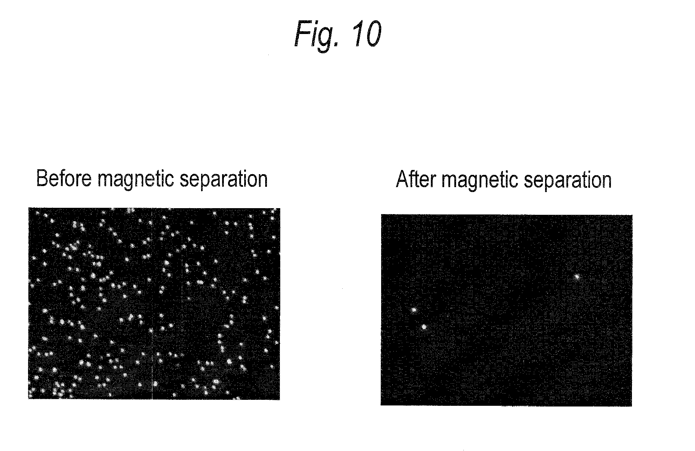

A magnetic particle having a gel layer was prepared in the same manner as in Example 1 and 50 µl of the obtained particle was added to 100 µl of FITC-labeled particles and stirred by rotation for 15 minutes. Subsequently, the obtained solution was subjected to magnetic separation and the supernatant was removed. To the residue, 1 ml of PBS was added and then the magnetic particles were redispersed. After magnetic separation once more, the supernatant was removed and the magnetic particles were redispersed in 1 ml of PBS. The same operation was repeated twice more. Subsequently, the obtained solution was observed using a fluorescent microscope. The results are shown in Fig. 10. As shown in Fig. 10, it is found that little non-specific adsorption due to the magnetic particles of the invention exhibited.

From these results, it is found that the intended substance was specifically separated using the magnetic particle to which an antibody specific to the intended substance to the magnetic particle of the invention was immobilized.

### Example 9:

### <Comparison with commercially-available magnetic particles in selection with particles>

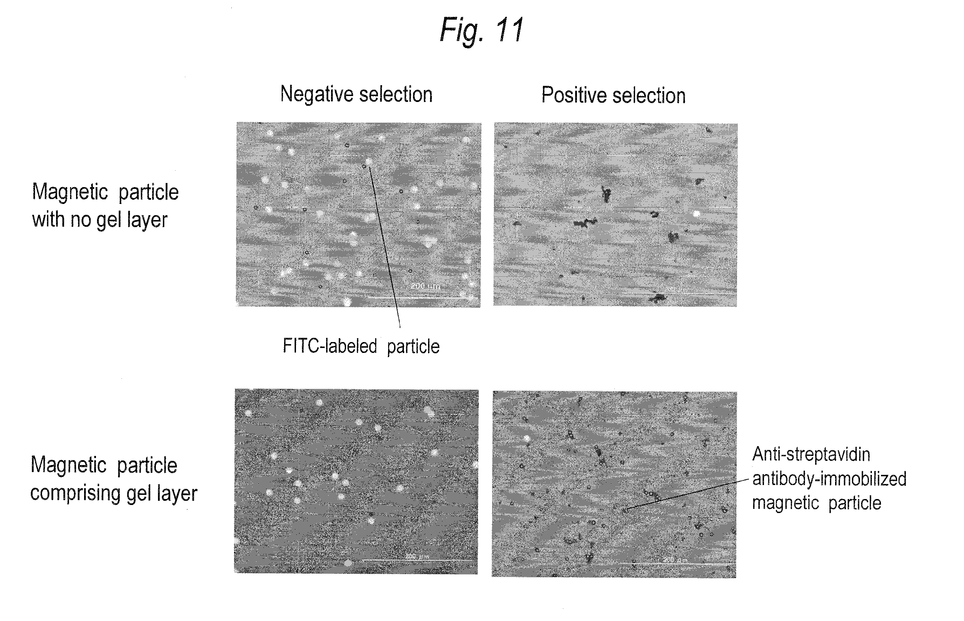

A commercially-available magnetic particle (Dynabeads MyOne Carboxylic Acid, manufactured by Invitrogen) (hereinafter, referred to as "magnetic particles with no gel layer") was compared with the anti-streptavidin antibody-immobilized magnetic particle comprising a gel layer prepared in Example 8. In the same manner as in Example 8, the magnetic particles were added to a mixture of FITC-labeled particles and streptavidin-labeled particles and then subjected to magnetic separation. After the first magnetic separation, the side of supernatant was sampled as a supernatant. (sup) sample; and the side of the magnetic particles was as a precipitation (ppt) sample. These samples were observed using a fluorescent microscope and the results are shown in Fig. 11.

As shown in Fig. 11, it is found that the magnetic particle with no gel layer failed in efficient selection, but the anti-streptavidin antibody-immobilized magnetic particle comprising a gel layer attained efficient selection of particles in that almost all the FITC-labeled particles were separated in the sup sample side while the streptavidin-labeled particles were in the ppt sample side.

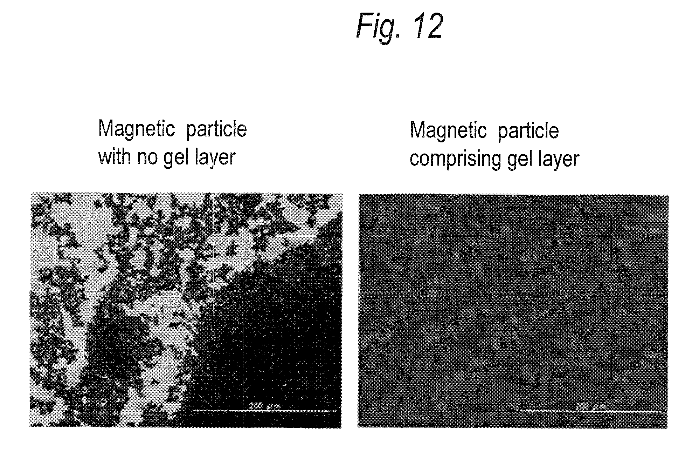

A mixture prepared by mixing 5 µl of the magnetic particles with no gel layer or the magnetic particles comprising a gel layer and 5 µl of streptavidin-labeled particles were observed with a microscope. The results are shown in Fig. 12.

As shown in Fig. 12, it is known that when the anti-streptavidin antibody-immobilized magnetic particles comprising a gel layer and streptavidin-labeled particles exist together, the streptavidin-labeled particles were readily distinguished. On the other hand, when the magnetic particles with no gel layer and streptavidin-labeled particles exist together, the streptavidin-labeled particles were hardly distinguished.

From these results, it is found that the magnetic particles comprising a gel layer of the invention can more specifically separate the intended substance than the commercially-available magnetic particles with no gel layer.

### Example 10

### <Magnetic separation from concentrated tap water and concentrated river water>

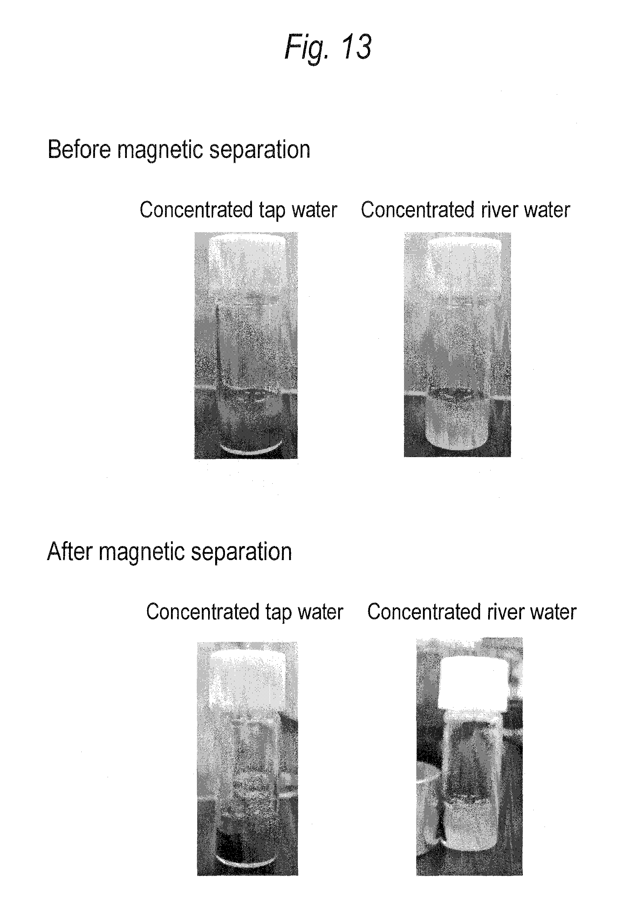

Each 1 ml of concentrated tap water (10 liters of tap water was concentrated to 10 ml) or concentrated river water [10 liters of river water (sampling point, Yoro River of Chiba Prefecture; sampling time, September to October 2008) was concentrated to 10 ml) was put into a 2-ml vial, and 100 µl of the magnetic particle comprising a gel layer which was prepared in the same manner as in Example 1 was added to each vial. Subsequently, for magnetic separation, a neodymium magnet (NE001Φ8×3, round magnet manufactured by Niroku Seisakusho) was attached to the side of the vial and left to stand for 1 minute. The behavior of magnetic separation was confirmed. The results are shown in Fig. 13.

As shown in Fig. 13, the magnetic particle comprising a gel layer of the invention was magnetically separated from both the concentrated tap water and the concentrated river water with little inclusion in the water.

### Example 11:

### <Selection of Giardia and cryptosporidium>

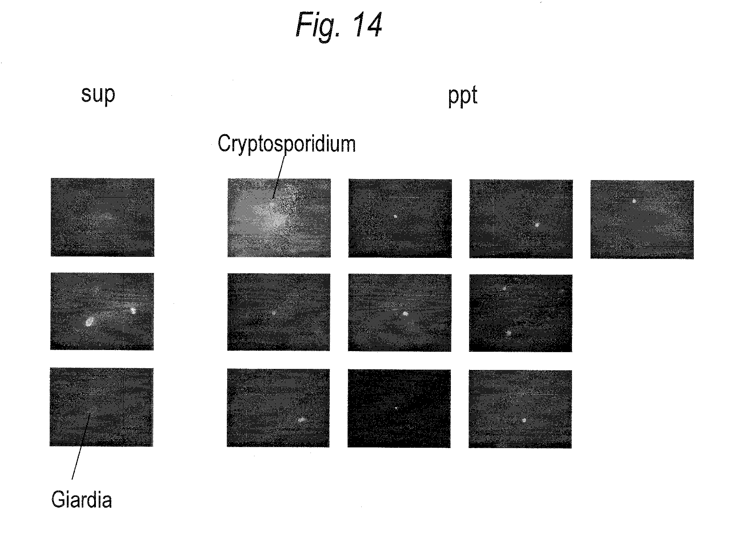

Each suspension containing 100 cells of inactivated Giardia or cryptosporidium; was added to 1 ml of the concentrated river water prepared in the same manner as in Example 10. To 1 ml of the suspension, 10 µl of biotinated cryptosporidium antibody (A400BIOT-R-20X: Crypt-a-Glo, manufactured by Waterborne) was added and then stirred and reacted by rotation for 30 minutes. Subsequently, to the obtained solution, 50 µl of the streptavidin-immobilized magnetic particles prepared in the same manner as in Example 2 were added, and mixed and reacted by shaking for 15 minutes. After magnetic separation for 2 minutes, the supernatant was collected to be a supernatant (sup) sample. To the separated magnetic particles, 1 ml of PBS was added and then the magnetic particles were redispersed. After magnetic separation, the supernatant was removed. To the residue, 200 µl of PBS was added and the magnetic particles were dispersed to be a precipitation (ppt) sample. The sup sample and the ppt sample were stained with EasyStain^{™} (manufactured by Wako Pure Chemical) and observed with a fluorescent microscope. The results are shown in Fig. 14.

As shown in Fig. 14, only Giardia was found in the sup sample and only cryptosporidium was found in the ppt sample. Since the magnetic particle of the invention does not comprise styrene in the particle composition, the magnetic particle exhibits no autofluorescence. Therefore, the fluorescence assay could be carried out without separation operation from the magnetic particles. From these results, it is found that, when the magnetic particles of the invention are used, cryptosporidium which could be hardly separated using the magnetic particles used in the official method can be separated from river water.

### Example 12:

### <Influence of crosslinking monomer>

### (Preparation of protein A-immobilized magnetic particles)

As shown in Table 1, glycidyl methacrylate, poly(ethylene glycol) methyl ether methacrylate (having the number-average molecular weight of 2080), polyethylene glycol diacrylate (having the number-average molecular weight of 575) and 2,2'-azobisisobutyronitrile were added to 100 ml of a methanol solution of magnetite-sodium oleate composite (magnetic particles), at a compounding ratio as shown in Table 1. Each of the resultant was subjected to the same manner as in Example 1 to prepare a magnetic particle comprising a gel layer on the surface. The obtained magnetic particle was subjected to the same manner as in Example 3 to prepare protein A-immobilized magnetic particle.

**[Table 1]**

| No. | PEGdiAc (g) | PEGMe (g) | GMA (g) | AIBN (g) | OCM (g) | MeOH (g) |
|---|---|---|---|---|---|---|
| 1 | 0.02 | 0.4 | 5.0 | 0.05 | 2.0 | 100 |
| 2 | 0.08 | 0.4 | 5.0 | 0.05 | 2.0 | 100 |
| 3 | 0.4 | 0.4 | 5.0 | 0.05 | 2.0 | 100 |
| 4 | 2.0 | 0.4 | 5.0 | 0.05 | 2.0 | 100 |
| 5 | 4.0 | 0.4 | 5.0 | 0.05 | 2.0 | 100 |

| | | | | | | |
|---|---|---|---|---|---|---|
| PEGdiAc: polyethylene glycol diacrylate PEGMe: poly(ethylene glycol) methyl ether methacrylate GMA: glycidyl methacrylate AIBN: 2,2'-azobisisobutyronitrile OCM: magnetite-sodium oleate composite MeOH: methanol | | | | | | |

The particle size, the water content, the concentration of epoxy group and the protein A immobilization degree of the thus-obtained, protein A-immobilized magnetic particles were measured. The data are shown in Table 2.

**[Table 2]**

| No. | Particle Size (nm) | Water Content (%) | Epoxy Group Concentration (10⁻⁷ mol/mg) | Protein A Immobilization Degree (µg/mg) |
|---|---|---|---|---|
| 1 | 537 | 83 | 10.1 ± 0.5 | 11.2 ± 1.5 |
| 2 | 577 | 70 | 8.1 ± 0.5 | 8.1 ± 0.5 |
| 3 | 536 | 65 | 7.4 ± 0.4 | 11.4 ± 1.2 |
| 4 | 3550 | 22 | 8.2 ± 0.2 | 5.6 ± 1.5 |
| 5 | 6440 | 15 | 8.7 ± 0.3 | 3.6 ± 1.1 |

As shown in Table 2, although the epoxy group content of all the particles are nearly on the same level, the magnetic particles having a smaller crosslinking monomer amount (Nos. 1 to 3) had a higher protein A immobilization degree than the magnetic particles having a larger crosslinking monomer amount (Nos. 4 and 5). It was considered that this was because the water content based on the crosslinking density of the particles would have a significant effect on the protein A immobilization in the particles.

### (Isolation of IgG)

In the same manner as in Example 4, IgG was magnetically separated using the protein A-immobilized magnetic particles. The IgG isolation was determined by SDS-PAGE electrophoresis. The results are shown in Table 3. In Table 3, the IgG isolation (mg/mg) is the amount of the isolated IgG per mg by the protein A-immobilized magnetic particles.

**[Table 3]**

| No. | IgG isolation (mg/mg) |
|---|---|
| 1 | 0.11 |
| 2 | 0.10 |
| 3 | 0.10 |
| 4 | 0.06 |
| 5 | 0.03 |

As in Table 3, it was found that the IgG isolation using the protein A-immobilized magnetic particles depended on the protein A immobilization degree of the particles.
While the invention has been described in detail and with reference to specific embodiments thereof, it will be apparent to one skill in the art that various changes and modifications can be made therein without departing from the spirit and scope thereof.
This application is based on Japanese application No. 2009-27896, filed on December 8, 2009, and Japanese application No.2010-230735, filed on October 13, 2010, the entire contents of which are incorporated hereinto by reference. All references cited herein are incorporated in their entirety.

## Claims

1. A magnetic particle, comprising an aggregate which comprises a magnetic substance and a compound having an alkyl group, and a gel layer which covers the aggregate, wherein the gel layer has a hydrophilic group.

2. The magnetic particle according to claim 1, having a mean particle size of from 0.9 nm or more to less than 10000 nm.

3. The magnetic particle according to claim 1 or 2, wherein the compound having an alkyl group is a compound which does not have a benzene ring and can bind to an iron ion.

4. The magnetic particle according to claim 3, wherein the compound having an alkyl group is a fatty acid.

5. The magnetic particle according to claim 4, wherein the fatty acid is oleic acid.

6. The magnetic particle according to any one of claims 1 to 5, wherein the hydrophilic group is an epoxy group.

7. The magnetic particle according to any one of claims 1 to 6, wherein the gel layer is a crosslinked body obtained from a polymerizable monomer and a crosslinking monomer.

8. The magnetic particle according to claim 7, wherein the polymerizable monomer is glycidyl methacrylate and poly(ethylene glycol) methyl ether methacrylate, and the crosslinking monomer is polyethylene glycol diacrylate.

9. The magnetic particle according to any one of claims 1 to 8, which does not emit autofluorescence,

10. An adsorption material comprising the magnetic particle of any one of claims 1 to 9, and a substance having an affinity to the intended substance.

11. The adsorption material according to claim 10, wherein the substance having an affinity to the intended substance is avidin, protein A or antibody.

12. A method for separating an intended substance, comprising at least the following steps (1) and (2) and uses the adsorption material according to claim 10 or 11:
(1) a step of preparing a mixed solution of an aqueous solution containing the intended substance and the adsorption material to form a combined body of the intended substance and the adsorption material,
(2) a step of separating the combined body by a magnetic force from the mixed solution obtained in the step (1).

13. The method according to claim 12, further comprising the following step (3):
(3) a step of eluting the intended substance from the combined body separated in the step (2).

14. A method of wastewater treatment, comprising using the method according to claim 12 or 13.

15. The wastewater treatment method according to claim 14, wherein at least one of cryptosporidium and Giardia are separated from the wastewater.

16. A method for producing magnetic particle, comprising at least the following steps:
(1) a step of forming an aggregate that contains a magnetic substance and an compound having an alkyl group,
(2) a step of forming a gel layer having a hydrophilic group on the surface of the aggregate formed in the step (1) by mixing a polymerizable monomer and a crosslinking monomer and then carrying out polymerization and crosslinking.
